(19) Europäisches Patentamt
European Patent Office
Office européen des brevets

(11) Publication number: 0 008 152
B1

(12) # EUROPEAN PATENT SPECIFICATION

(45) Date of publication of patent specification: 18.01.84

(51) Int. Cl.$^3$: A 61 L 17/00 //C08G63/66

(21) Application number: 79300252.8

(22) Date of filing: 20.02.79

(54) **Non-absorbable synthetic sutures and ligatures.**

(30) Priority: 14.08.78 US 933224

(43) Date of publication of application:
20.02.80 Bulletin 80/4

(45) Publication of the grant of the patent:
18.01.84 Bulletin 84/3

(84) Designated Contracting States:
BE CH FR GB IT NL SE

(56) References cited:
GB - A - 1 282 745
US - A - 3 942 532

(73) Proprietor: AMERICAN CYANAMID COMPANY
Berdan Avenue
Wayne New Jersey 06904 (US)

(72) Inventor: Kaplan, Donald Samuel
20 Ritch Drive
Ridgefield Connecticut 06877 (US)

(74) Representative: Allam, Peter Clerk et al,
LLOYD WISE, TREGEAR & CO. Norman House
105-109 Strand
London WC2R 0AE (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

# Non-absorbable synthetic sutures and ligatures

This invention relates to a sterile surgical suture or ligature, which can be a monofilament, or a twisted or braided multifilament article.

The medical profession is continuously seeking more satisfactory sutures to be used in closing wounds, whether such wounds are incisions from operations, or tears, cuts or abrasions from accidental or other causes. Many materials have been suggested for use as sutures. Sutures are divided into two broad classes, the absorbable sutures, such as catgut or polyglycolic acid sutures, which are absorbed by the body tissues, and nonabsorbable sutures which either remain in the tissues in substantially their original form for prolonged periods or are removed from the skin surfaces after the underlying tissues have been healed. For non-absorbable sutures many materials have been suggested which range from cotton and silk through various synthetic filaments such as polypropylene to stainless steel or nickel or other metallic filaments.

Other things being equal, the medical profession usually prefers the suture which is strongest. In spite of many disadvantages stainless steel has met with considerable acceptance because of its extremely high tensile strength. Such plastics material as polypropylene are meeting currently with considerable commercial acceptance because of comparatively high tensile strength and because of other advantages over stainless steel.

Additionally, the suture material needs good handling characteristics. The handling characteristics of a suture, as a general statement, are difficult to define but should include a high degree of flexibility. Handling characteristics also include knot strength and knot security; that is, the suture must have such characteristics that a knot can be tied in the suture. Some materials are so brittle that if a suture made from them is knotted, the strength of the suture is markedly reduced. For some materials an over-hand knot in a strand can reduce the strength of the strand by a factor of two or more. In addition to knot strength, the suture should have such characteristics that the knot when tied remains in position. Also, the suture should be "throwable" so that when the free end is placed in position by the surgeon it will remain in that position until moved. Similarly, the suture should have such characteristics that it can be thrown or moved from side to side and yet retain the position into which it is thrown.

A surgical suture comprising a high degree of tensile strength with a high degree of flexibility is therefore needed in the medical profession.

A polypropylene monofilament suture is one attempt at solving this need. The tensile strength of this suture is good when compared to stainless steel; however the flexibility of the suture, though better than stainless steel, is still considered to be stiff and springy.

A polyurethane suture is another attempt. The primary advantage of this suture is its very high degree of flexibility. However, polyurethane sutures have low tensile strength and extremely high elongations at break which make then unsatisfactory for general wound closure methods.

Other attempts include the braiding of materials with a high tensile strength but a low degree of flexibility. Polyethylene terephthalate is an example of a suture material with a satisfactory tensile strength after braiding and an increased degree of flexibility. A monofilament suture is generally preferred in most surgical procedures to a braided suture because of the reduced tissue drag of the monofilament. Also, in skin suturing a monofilament suture is generally preferred because it is usually less susceptible to capillary action than a braided suture.

The present invention provides a non-absorbable sterile surgical suture or ligature comprising a filament formed from a block copolymer containing polymeric blocks (A) and polymeric blocks (B), wherein the polymeric blocks (A) consist of a polyalkylene ether unit having a number average molecular weight of from 500—3000 and have the formula:

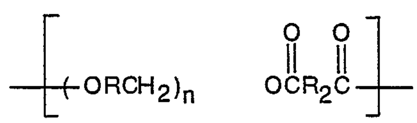

wherein R is a straight or branched chain alkylene group of from 1 to 9 carbon atoms, $R_2$ is 1,4-phenylene or cyclohexylene and n is the number of repeating units; and wherein the polymeric blocks (B) are the reaction product of (i) an aromatic dicarboxylic acid or a cycloaliphatic dicarboxylic acid and (ii) a short chain aliphatic or cycloaliphatic diol, and have the formula:

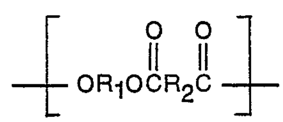

wherein $R_1$ is a straight or branched chain alkylene group of from 2 to 10 carbon atoms or a cyclic group having the formula:

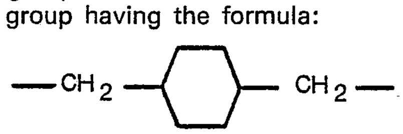

and $R_2$ is 1,4-phenylene or cyclohexylene; said polymeric blocks (B) comprising from 30% to 95% by weight of said copolymer; and said copolymer having a number average molecular weight of from 25,000 to 30,000.

A surgical suture or ligature wherein the polymeric blocks (B) comprise from 50% to 85% of the copolymer is preferred, with the most preferred content of blocks (B) being from 55% to 80%.

A surgical suture or ligature described above wherein R is selected from ethylene, propylene and butylene groups is also preferred, and where R is a propylene group is most preferred.

Within the scope of this invention is a surgical suture or ligature described above and having an attached needle.

As noted above, the suture or ligature of this invention may be in monofilament form or it may be of a braided or twisted construction. For reasons already explained, monofilament sutures and ligatures are however preferred. By means of this invention it is possible, in preferred embodiments, to provide a monofilament suture or ligature which has good flexibility and fatigue life and high tensile strength. More particularly, we can provide a monofilament suture or ligature which has approximately the following characteristics:

| | |
|---|---|
| Straight Pull | At least 3515 kg/cm² (50,000 p.s.i.) |
| Knot Pull | At least 2461 kg/cm² (35,000 p.s.i.) |
| Flexual Modulus | Less than 24610 kg/cm² ($3.5 \times 10^5$ p.s.i.) |
| Flexual Fatigue, cycles to failure | At least 1,000 |
| Elongation at break, percent | Less than 100% |
| Draw ratio | Between 5 x and 10 x |

A surgical suture or ligature wherein the elongation at break precent is between 25 and 55 is preferred.

Also within the scope of this invention is a surgical suture package comprising a sterile enclosure and within which is a non-absorbable sterile surgical suture or ligature of this invention, as described above.

Preferred monofilament sutures and ligatures of this invention combine the advantages of a braided suture material (i.e. flexibility and knot security) with those of a monofilament suture material (i.e. smooth surface, low tissue drag, inertness, and ease of knot run down).

The structures of the block copolymers used in this invention may be represented by the following general formula:

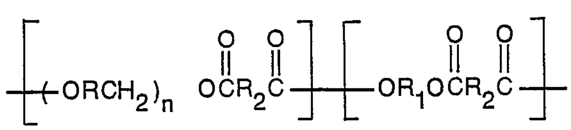

(A) (B)

wherein R is a straight or branched chain alkylene group of 1 to 9 carbon atoms; $R_1$ is a straight or branched chain alkylene group of from 2 to 10 carbon atoms or a cyclic group having the formula:

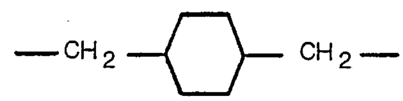

$R_2$ is selected from phenylene and cyclohexylene; and n is the number of repeating units.

In the polymeric blocks A, for example, units derived from polyethylene oxide or polybutylene oxide may constitute the soft segment of the block copolymer.

In the polymeric blocks B, for example, units derived from polyethylene terephthalate or polybutylene terephthalate may constitute the hard segment of the block copolymer.

In order to have the desired qualities of flexibility and high tensile strength, the sutures and ligatures of this invention must be formed from a copolymer containing polymeric blocks A and B, wherein the hard segment, provided by the polymeric blocks B, constitutes 30—95% by weight of the copolymer, and as already noted the B component should preferably constitute 50—85% by weight, most preferably 55—80% by weight, of the copolymer.

Preferably, the soft segment, provided by the polymeric blocks A, is derived from a (tetramethylene ether) glycol having a number average molecular weight in the range of 500—3000. The total number average molecular weight of the block polyether-esters i.e. the copolymer is 25—30,000.

The hard segment, provided by polymeric blocks B, can be derived from (1) a diacid, for example,

(a) terephthalic acid,

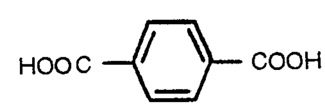

or,

(b) 1,4-cyclohexane dicarboxylic acid; or

(c) from the dimethyl esters of these acids; and,

(2) A short chain glycol, for example,

(a) a linear or branched chain glycol of 2 to 10 carbon atoms; and preferably of 4 carbon atoms, or

(b) 1,4-cyclohexanedimethanol,

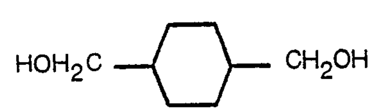

The reaction product of (1) and (2) forms the polymeric blocks B.

Methods for preparing these block

copolymers are known in the art. See, e.g., Belgium Patent 832,445 issued December 1, 1975; German Offen. 2,265,320 published May 6th, 1977 and German Offen. 2,265,294 published April 21, 1977.

Sutures and ligatures can be formed from the block copolymers described in this application by conventional extrusion and drawing techniques. Generally, the extruded filaments are drawn from 5 to 10 times the original length of the undrawn extruded strand, preferably from 6 to 8 times, and this draw is preferably effected in two stages. If desired, the drawn filament may be subjected to conventional post-treatments, e.g. heating at constant length at, say, about 185°C.

The sutures and ligatures formed from the block copolymers in accordance with this invention can be sterilized by a variety of methods recognized in the art including exposure to a gaseous sterilizing agent such as ethylene oxide, and exposure to radiation of gamma rays. However, the sutures of this invention cannot generally be sterilized by exposure to heat because the flexible properties of the sutures may be adversely affected.

The sutures of this invention can, if desired, be colored by mechanically blending with a pigment. Pigments such as titanium dioxide, iron oxide or carbon black give identifiable colors. Other colored pigments which do not cause deleterious tissue reactions may also be used to impart color to the strands.

Sutures formed from the block copolymers in accordance with this invention were tested for toxicity by placing two 4 cm. segments on each of three plates of HEp-2 cell culture. The cultures were incubated for 24 hours at 36°C., stained with crystal violet and checked for degeneration of the cell monolayer in the area of the suture segment. None of the suture segments tested showed any evidence of cytotoxicity.

The sutures were also tested in mice by the Systemic Injection Test according to the U.S.P. Biological Test for Plastic Containers No. . The sutures were subjected to an extraction procedure and the extractant was injected into mice. No adverse reactions were observed in any of the test animals.

For use as sutures, and ligatures, any size may be used, depending upon the preference of the surgeon. In the United States, the more common standard sizes are defined in the United States Pharmacopeia (which is abbreviated U.S.P.) as follows: (see United States Pharmacopeia Convention, Inc., Mack Publishing Co., Easton, Pa.):

| U.S.P. Size | U.S.P. Diameter (inches max.) |
|---|---|
| 6—0 | 0.004 (or 0.1016 mm) |
| 5—0 | 0.006 (or 0.1524 mm) |
| 4—0 | 0.008 (or 0.2032 mm) |
| 3—0 | 0.010 (or 0.254 mm) |
| 00 | 0.013 (or 0.3302 mm) |
| 0 | 0.016 (or 0.4064 mm) |

The results of tests conducted to compare three types of non-absorbable sutures: (1) Dermalon®, a nylon suture manufactured by American Cyanamid Company, Wayne, New Jersey; and (2) Surgilene®, a polypropylene suture of American Cyanamid Company, Wayne, New Jersey, and (3) the copolymers of this invention are disclosed in the following examples.

Example 1

A copolyester composed of a polytetramethylene oxide soft segment (MW = 1000) and a poly(tetramethylene terephthalate) hard segment and containing 58 ± 2% by weight of the hard segment is extruded at 230°—245°C. with a collection of extrudate at about 7.625 metres (25 feet) per minute. A two stage draw was used an 8X draw at 160°C in zone 1 and 1.1X draw at 120°C in zone 2 for a total draw of 8.8X (where X is the original length of the undrawn strand). The properties of this size 3/0 USP fiber (0.245 mm) are described in the Table below.

Example 2

A copolyester composed of the hard and soft segments of Example 1 but with a content of 76% by weight hard segment is extruded at 230°C—245°C. A two stage draw is used where zone 1 is at 165°C. and drawn 2X and zone 2 is drawn 3.5X at 150°C. for a total draw of 7.0X. The properties of this size 3/0 U.S.P. fiber (0.231 mm) are described in the Table below.

Example 3

A copolyester composed of the hard and soft segments of Example 1, but with a content of 66% by weight hard segment is extruded at 230°—245°C. A two stage draw is used where Zone 1 is at 160°C (320°F) and drawn 2X and Zone 2 is at 160°C (320°F) and drawn at 4.1 for a total draw of 8.2X. The fiber is then heated at constant length at 185°C for 10 minutes. The properties of this fiber (0.195 mm diameter) are described in the Table below.

TABLE

| | STRAIGHT PULL [1] | KNOT PULL [1] | FLEXUAL MODULUS [2] | FLEXUAL FATIGUE [3] | ELONGATION AT BREAK | TENACITY [4] |
|---|---|---|---|---|---|---|
| Example 1 Copolymer | 75,256 (5291) | 41,055 (2886) | $0.54 \times 10^5$ (3800) | 12,972 | 53% | 4.6 |
| Example 2 Copolymer | 80,049 (5628) | 38,485 (2706) | $1.31 \times 10^5$ (9210) | 6,665 | 36% | 4.6 |
| Example 3 Copolymer | 51,600 (3628) | 47,348 (3329) | $0.99 \times 10^5$ (6960) | – | 49% | 4.6 |
| DERMALON® | 70,200 (4936) | 49,400 (3473) | $6.45 \times 10^5$ (45350) | 519 | 37% | 4.2 |
| SURGILENE® | 64,000 (4500) | 45,000 (3164) | $9.98 \times 10^5$ (70170) | 807 | 24% | 5.3 |

1 pounds per square inch, ASTM D2256 — values in brackets are $kg/cm^2$

2 pounds per square inch, ASTM D790 — values in brackets afe $kg/cm^2$

3 Cycles to failure, Folding Endurance Tester, Tinius Olsen Co.

4 Grms./Denier, ASTM D2256.

## Claims

1. A non-absorbable sterile surgical suture or ligature comprising a filament formed from a block copolymer containing polymeric blocks (A) and polymeric blocks (B), wherein the polymeric blocks (A) consist of a polyalkylene ether unit having a number average molecular weight of from 500—3000 and have the formula:

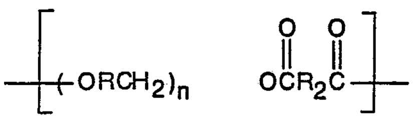

wherein R is a straight or branched chain alkylene group of from 1 to 9 carbon atoms, $R_2$ is 1,4-phenylene or cyclohexylene and n is the number of repeating units; and wherein the polymeric blocks (B) are the reaction product of (i) an aromatic dicarboxylic acid or a cycloaliphatic dicarboxylic acid and (ii) a short chain aliphatic or cycloaliphatic diol, and have the formula:

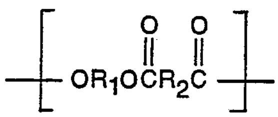

wherein $R_1$ is a straight or branched chain alkylene group of from 2 to 10 carbon atoms or a cyclic group having the formula:

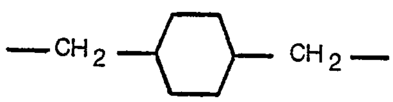

and $R_2$ is 1,4-phenylene or cyclohexylene; said polymeric blocks (B) comprising from 30% to 95% by weight of said copolymer; and said copolymer having a number average molecular weight of from 25,000 to 30,000.

2. A surgical suture or ligature according to Claim 1, wherein the polymeric blocks (B) comprise from 50% to 85% by weight of the copolymer.

3. A surgical suture or ligature according to Claim 2, wherein the polymeric blocks (B) comprise from 55% to 80% by weight of the copolymer.

4. A surgical suture or ligature according to any preceding claim, wherein R is selected from ethylene, propylene and butylene.

5. A surgical suture or ligature according to Claim 4, wherein R is propylene.

6. A surgical suture or ligature according to any preceding claim in monofilament form.

7. A surgical suture or ligature according to any preceding claim and having an attached needle.

8. A surgical suture or ligature according to any preceding claim which has been annealed by being heated at constant length.

9. A surgical suture package comprising a sterile enclosure within which is a non-absorbable monofilament sterile surgical suture or ligature according to any preceding claim.

## Revendications

1. Suture ou ligature chirurgicale stérile non absorbable comprenant un bloc polymère (A) constitué d'une unité d'éther polyalkylénique de formule

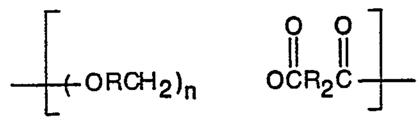

ayant une moyenne numérique du poids moléculaire d'environ 500 à 3000, dans laquelle R représente un radical alkyle droit ou ramifié ayant environ 1 à 9 atomes de carbone et $R_2$ représente un radical phénylène-1,4 ou cyclohexylène et n est le nombre des motifs, et un bloc polymère (B) qui est le produit de la réaction d'un acide dicarboxylique aromatique ou d'un acide cycloaliphatique, et d'un diol aliphatique ou cycloaliphatique à chaîne courte de formule

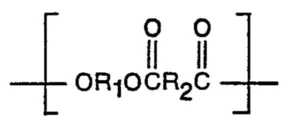

dans laquelle $R_1$ représente un radical alkyle droit ou ramifié ayant environ 2 à 10 atomes de carbone ou un radical cyclique de formule:

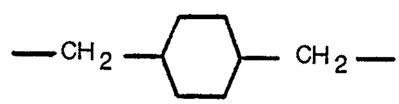

et $R_2$ représente un radical phénylène-1,4 ou cyclohexylène; ledit bloc (B) constituant environ 30 à 95% dudit copolymère; et ledit copolymère ayant une moyenne numérique du poids moléculaire d'environ 25 000 à 30 000.

2. Suture ou ligature chirurgicale selon la revendication 1, dans laquelle le bloc polymère (B) constitue environ 50 à 85% du copolymère.

3. Suture ou ligature chirurgicale selon la revendication 2, dans laquelle le bloc polymère (B) constitue environ 55% à 85% du copolymère.

4. Suture ou ligature chirurgicale selon l'une quelconque des revendications précédentes, dans laquelle R est choisi parmi l'éthylène, le propylène et le butylène.

5. Suture ou ligature chirurgicale selon la revendication 4, dans laquelle R représente un radical propylène.

6. Suture ou ligature chirurgicale selon l'une quelconque des revendications précédentes sous forme d'un monofilament.

7. Suture ou ligature selon l'une quelconque

des revendications précédentes, à laquelle est fixée une aiguille.

8. Suture ou ligature chirurgicale selon l'une quelconque des revendications précédentes, que l'on a recuite par chauffage à longueur constante.

9. Emballage contenant une suture chirurgicale comprenant une enveloppe stérile dans laquelle se trouve une suture ou ligature chirurgicale stérile monofilamentaire, non absorbable selon l'une quelconque des revendications précédentes.

**Patentansprüche**

1. Nicht-resorbierbares, steriles, chirurgisches Nahtmaterial oder Ligaturmaterial, umfassend ein Filament, welches erhalten wurde aus einem Block Copolymeren, das Polymerblöcke (A) und Polymerblöcke (B) enthält, wobei die Polymerblöcke (A) aus einer Polyalkylenäthereinheit mit einem Zahlenmittel des Molekulargewichts von 500 bis 3000 und der folgenden Formel bestehen

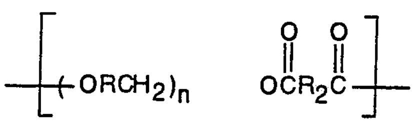

wobei R eine geradkettige oder verzweigtkettige Alkylengruppe mit 1 bis 9 Kohlenstoffatomen ist, wobei $R_2$ eine 1,4-Phenylen- oder Cyclohexylengruppe ist und wobei n die Zahl der sich wiederholenden Struktureinheiten bedeutet; und wobei die Polymerblöcke (B) das Reaktionsprodukt von (i) einer aromatischen Dicarbonsäure oder einer cycloaliphatischen Dicarbonsäure und (II) eines kurzkettigen aliphatischen oder cycloaliphatischen Diols sind und die folgende Formel haben

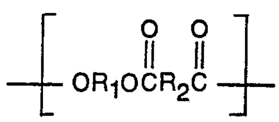

wobei $R_1$ eine geradkettige oder verzweigtkettige Alkylengruppe mit 2 bis 10 Kohlenstoffatomen bedeutet oder eine cyclische Gruppe der Formel

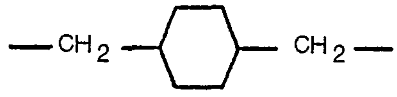

und wobei $R_2$ für 1,4-Phenylen- oder Cyclohexylen steht und wobei die Polymerblöcke (B) 30 bis 95 Gew.-% des Copolymeren ausmachen und wobei das Copolymere ein Zahlenmittel des Molekulargewichts von 25000 bis 30000 hat.

2. Chirurgisches Naht- oder Ligaturmaterial nach Anspruch 1, dadurch gekennzeichnet, daß der Polymerblock (B) etwa 50 bis 85% des Copolymeren ausmacht.

3. Chirurgisches Naht- oder Ligaturmaterial nach Anspruch 2, dadurch gekennzeichnet, daß der Polymerblock (B) etwa 55 bis 80% des Copolymeren ausmacht.

4. Chirurgisches Naht- oder Ligaturmaterial nach einem der vorhergehenden Ansprüche, dadurch gekennzeichnet, daß R ausgewählt ist aus Äthylen, Propylen und Butylen.

5. Chirurgisches Naht- oder Ligaturmaterial nach Anspruch 4, dadurch gekennzeichnet, daß R Propylen ist.

6. Chirurgisches Naht- oder Ligaturmaterial nach einem der vorhergehenden Ansprüche, dadurch gekennzeichnet, daß es in Monofilamentform vorliegt.

7. Chirurgisches Naht- oder Ligaturmaterial nach einem der vorhergehenden Ansprüche, dadurch gekennzeichnet, daß es mit einer Nadel verbunden ist.

8. Chirurgisches Naht- oder Ligaturmaterial nach einem der vorhergehenden Ansprüche, dadurch gekennzeichnet, daß es durch Erhitzen bei konstanter Länge getempert wurde.

9. Chirurgische Nahtmaterialpackung mit einer sterilen Umhüllung, in der ein nicht-resorbierbares, steriles, chirurgisches Nahtmaterial oder Ligaturmaterial in Monofilamentform nach einem der vorhergehenden Ansprüche untergebracht ist.